# EUROPÄISCHE PATENTSCHRIFT

(11) **EP 0 430 270 B1**
(45) Veröffentlichungstag und Bekanntmachung des Hinweises auf die Patenterteilung: **31.01.1996**
(21) Anmeldenummer: 90122908.8
(22) Anmeldetag: 30.11.1990
(51) Int. Cl.: C12N 1/08, C12N 1/14, C12N 9/22, C12N 11/00

(54) **Verfahren zur biologischen Inaktivierung von Nukleinsäuren**
Method for the biological inactivation of nucleic acids
Procédé pour l'inactivation biologique des acides nucléiques

(30) Priorität: 01.12.1989 DE 3939771
(43) Veröffentlichungstag der Anmeldung: 05.06.1991
(73) Patentinhaber: BEHRINGWERKE Aktiengesellschaft, D-35001 Marburg (DE)
(72) Erfinder: Bröker, Michael, Dr., W-3550 Marburg (DE); Fibi, Mathias, Dr., W-3550 Marburg (DE)

(56) Entgegenhaltungen:
- EP-A- 0 164 573
- EP-A- 0 229 866

## Beschreibung

Die Erfindung betrifft Verfahren zur biologischen Inaktivierung von Nukleinsäuren. Das aus biotechnologischen Produktionsprozessen anfallende, mit den verwendeten gentechnisch manipulierten Mikroorganismen kontaminierte Material (restliche Fermentationsbrühe, Spülwasser etc.) wird in Behältern gesammelt und durch Autolyse von und/oder gezielte Kontamination mit Paecilomyces liliacinus spp. abgebaut.

Biotechnologische Produktionsverfahren werden zunehmend eingesetzt, um biologische Substanzen und Wirkstoffe (Proteine, Polysaccharide, Antibiotika, Aminosäuren, Vitamine, Alkohole u.a.) zu gewinnen. Durch molekularbiologische Techniken ist es nun möglich, Proteine in artfremden Organismen zu produzieren. Dabei können mikrobielle, pflanzliche, tierische und menschliche Gene in den jeweils anderen Wirtszellen exprimiert werden. Heutzutage werden vorwiegend Bakterien wie Escherichia coli, Bacillus subtilis und Streptomyces lividans und Hefen wie Saccharomyces cerevisiae, Pichia pastoris und Kluyveromyces lactis zur Synthese von Proteinen herangezogen. Aber auch tierische Zellen (z.B. Ovarzellen des chinesischen Hamsters [CHO-Zellen], Mauszellen [C127i-Zellen]) und menschliche Zellen werden zur heterologen Genexpression eingesetzt. Die Produktion der Wirkstoffe erfolgt bevorzugt in Fermentern, da hier die Synthese durch moderne Regeltechnik gesteuert werden kann. Die Produkte werden entweder aus den Zellen gewonnen oder sie werden in die Kulturbrühe ausgeschleust.

Allen Produktionsverfahren ist gemein, daß letztlich große Mengen an Zellmaterial anfallen, die u.a. hauptsächlich natürliche und hömologe Nukleinsäuren (DNA und RNA) aber auch rekombinante Plasmid-DNA oder in das Genom der Wirtszelle integrierte Fremd-DNA enthalten. Eine Einschätzung möglicher Risiken bei der Freisetzung dieser rekombinanten DNA für die Umwelt ist bisher nur schwer möglich. Nach dem Vorsorgeprinzip wurden deshalb in den meisten Staaten gesetzliche Bestimmungen und Vorschriften von Behörden und Institutionen (wie z.B. in der Bundesrepublik Deutschland die Zentrale Kommission für biologische Sicherheit (ZKBS) des Bundesgesundheitsamtes in Berlin) erlassen, die nach dem Produktionsprozeß als Schutz vor einer möglichen Umweltgefährdung eine Inaktivierung des in der Regel flüssigen Abfallmaterials und damit der vorhandenen Nukleinsäuremenge vorschreiben.

Die Inaktivierung der Nukleinsäuren kann physikalisch, z.B. durch Hitze, erreicht werden. Dies ist ein Prozeß, der viel Energie erfordert und somit ein kostenintensives Verfahren darstellt. Andererseits,kann man die Nukleinsäuren chemisch inaktivieren. So wird z.B. in der Patentanmeldung WO 89/03226 ein Verfahren vorgestellt, anhand dessen DNA durch Hitzebehandlung in Gegenwart von Percarbonsäure, Alkaliperoxid oder Alkaliperoxomonosulfat chemisch inaktiviert wird. Ein ähnliches Inaktivierungsverfahren beruht auf der Verwendung von Zitronensäure. Dieses wie auch andere chemische Verfahren haben den Nachteil, daß sie für die Beschäftigten potentielle gesundheitliche Gefahren darstellen und/oder die verbleibenden Endprodukte umweltschädlich sind. In beiden Verfahren kann durch Zusatz der entsprechenden Chemikalien eine vollständige Inaktivierung der Nukleinsäure auch mit niedrigerer Temperatur erreicht werden (z.B. 80°C).

Als vorteilhaft sind demgegenüber solche Verfahren anzusehen, mit denen Nukleinsäuren bei Raumtemperatur biologisch inaktiviert werden können. Diese Verfahren sind effizient, kostengünstig und umweltverträglich. Sie basieren darauf, daß Nukleinsäuren durch spezielle Enzyme (Nukleasen, d.h. DNasen und RNasen) abgebaut werden und daß diese Enzyme u.a. durch Mikroorganismen gebildet werden, die in Kulturbrühe bzw. Abwasser vorhanden sind oder zugesetzt werden. In der EP-A-0 229 866 wird die Verwendung einer Nuklease aus Serratia spp. zur Entfernung von kontaminierenden Nukleinsäuren aus biologischem Material beschrieben.

Die Aufgabe der vorliegenden Erfindung bestand in der Bereitstellung eines einfachen Verfahrens zur Verwendung von Mikroorganismen zum Abbau von Nukleinsäuren in Fermentationsbrühen und Abwässern, in denen durch Lyse von Animalzellen DNA-Moleküle einschließlich rekombinanter DNA - freiwerden können.

Gelöst wird die Aufgabe durch ein Verfahren, bei dem das anfallende, mit Nukleinsäuren kontaminierte Material (restliche Förmentationsbrühe, Zellen, Spülwasser etc.) in Behältern gesammelt wird und nachdem es mit Paecilomyces liliacinus spp. angeimpft wurde, für einige Zeit unter Rühren bei Raumtemperatur inkubiert wird. Die in den Behälter gelangten DNasen (z.B. Freisetzung durch Zellyse oder durch Sekretion) können in kurzer Zeit DNA, einschließlich rekombinanter Plasmid-DNA, vollständig abbauen. Durch unvollständige Entleerung des Abwassersammelbehälters bleiben die mikrobiellen Populationen in ihrer Zusammensetzung erhalten. Sie können sich beim Wiederauffüllen des Behälters mit Abwasser weitervermehren und DNasen ausscheiden, wenn das Abwasser verwertbare Energiequellen wie zum Beispiel DNA enthält.

Somit werden im Abwasser befindliche rekombinante und auch wirtsspezifische Nukleinsäuren durch Nukleasen vollständig abgebaut und es erübrigt sich eine thermische oder chemische Inaktivierung der Abwässer. Es wurde ferner gefunden, daß die Mikroorganismen aus dem Abwassersammelbehälter rekombinante DNA nicht aufnehmen bzw. daß die rekombinante DNA, welche in Abwesenheit der DNase-Aktivität im Abwasser stabil vorliegen müßte, in den o.g. Mikroorganismen weder exprimiert noch repliziert wird.

Das Wachstum der nukleinsäureabbauenden Mikroorganismen erfolgt schon bei Raumtemperaur und ohne fermentationübliche Bedingungen wie Sauerstoffzufuhr, Rühren der Kulturbrühe und andere Maßnahmen. Doch kann zur schnelleren Entwicklung der Mikroorganismen und zur Erlangung höherer Zelldichten das Abwasser beispielsweise auch bei Temperaturen, die über der üblichen Raumtemperatur liegen, fermentiert werden.

Die nukleinsäureabbauenden Mikroorganismen können auch außerhalb des Abwassers vermehrt und dem Abwasser zur Erhöhung der DNase-Aktivität vor dem Inkubationsschritt in konzentrierter Form zugesetzt werden.

Im weiteren kann man auch die Gene, die für die sekretierten DNasen kodieren, klonieren und zusätzlich zu dem gewünschten Produkt exprimieren, so daß schon während des Produktionsprozesses DNasen gebildet und ausgeschieden werden, die freie DNA in der Kulturbrühe und daraufhin im Abwasser abbauen.

Es können auch isolierte DNasen dem Abwasser zugesetzt werden. In einer besonderen Ausführungsform können die DNasen an ein Carriermaterial immobilisiert werden, wobei das Carriermaterial mit dem Zellcarriermaterial identisch sein kann.

Der enzymatische Abbau von rekombinanter DNA durch diese neu entwickelten Verfahren besitzt Vorteile gegenüber physikalischen oder chemischen Inaktivierungsverfahren von Nukleinsäuren. Diese Verfahren verzichten auf Agenzien, die für das beschäftigte Personl und für die Umwelt unverträglich sind. Sie sind wesentlich kostengünstiger, weniger arbeitsintensiv, weniger störanfällig und sind unter dem Gesichtspunkt des Arbeitsschutzes wesentlich sicherer als z.B. die Inaktivierung von Nukleinsäuren durch Erhitzen, insbesondere durch Autoklavieren oder durch Chemikalien. Eine Aufnahme rekombinanter DNA durch Mikroorganismen wurde nicht beobachtet, so daß es weder zur Replikation noch zur Expression der rekombinanten DNA kommt. Die rekombinante DNA wird vielmehr vollständig verstoffwechselt, d.h. rekombinante DNA wird durch dieses Verfahren in natürliche Biomasse umgewandelt, die sich in ihrer mikrobiellen Populationszusammensetzung von der Biomasse in natürlichen Biotopen oder Kläranlagen nicht wesentlich unterscheidet.

Die Erfindung betrifft folglich Verfahren zur Inaktivierung von Nukleinsäuren, dadurch gekennzeichnet, daß autolytisch und/oder sekretorisch freigesetzte RNasen und/oder DNasen von Paecilomyces liliacinus spp. einwirken. Bevorzugt sind Verfahren unter Verwendung von während der Fermentation unter sterilen Bedingungen freigesetzten natürlichen zellulären oder rekombinanten Nukleasen. Besonders bevorzugt sind Verfahren mit Zusatz von gereinigten Nukleasen oder nukleasefreisetzenden Paecilomyces liliacinus spp., ganz besonders mit dem Paecilomyces liliacinus AW13, hinterlegt nach Budapester Vertrag unter Nr. DSM 5650 bei der Deutschen Sammlung von Mikroorganismen. Ferner betrifft die Erfindung den o.g. Schimmelpilz Paecilomyces liliacinus AW13 selbst einschließlich der nukleaseausscheidenden anderen Stämme dieser Spezies.

Die Erfindung ist ferner in den Beispielen und den Patentansprüchen enthalten.

### Beispiele

Die nachfolgenden Beispiele beziehen sich auf die herkömmlichen Produktionsanlagen für rekombinantes, menschliches Erythropoetin (EPO). Die Produktionsanlage nach "klassischem" Prinzip besteht aus einem Fermenter, einem Abwasserauffangbehälter und einer Abtötungsanlage. Zellinhalt und Carriermaterial der Fermentationsbrühe werden durch Absetzen getrennt. Danach wird der Überstand abgezogen und durch Filtration geklärt. Dabei wird der Überstand nach der Filtration einem Reinigungsverfahren zur Gewinnung von menschlichem Erythropoetin unterworfen, während die festen Bestandteile, also Zell- und Carrierrückstände mit dem Filter durch Verbrennung entsorgt werden. Abfallflüssigkeiten aus dem Reinigungsverfahren werden in den Abwasserauffangbehälter geleitet. Nach Abziehen der Kulturbrühe wird das Zell- und Carriersediment mit Leitungswasser ausgespült und in den Abwasserauffangbehälter geleitet. Danach wird der Fermenter mit Leitungswasser gefüllt und 20 Minuten auf 120°C erhitzt. Nach Abkühlen auf 80°C wird dieses Wasser dann ebenfalls in den Abwasserauffangbehälter geleitet. Zusätzliches Abwasser, was bei der weiteren Reinigung des Fermenters anfällt, wird ebenfalls in den Abwasserauffangbehälter eingeleitet. Das Abwasser im Abwasserauffangbehälter setzt sich somit aus Abwasser aus dem Reinigungsverfahren für EPO und der Fermenterreinigung zusammen. Sämtliche Abwassergemische weisen aufgrund der Verwendung von Leitungswasser für die Fermenterreinigung für Animalzellen unphysiologische Bedingungen auf. Hat die Flüssigkeit im Abwasserauffangbehälter eine gewisse Füllhöhe erreicht, wird das Abwasser 30 Minuten lang gerührt und danach in die Abtötungsanlage gepumpt. In der Abtötungsanlage wird die Flüssigkeit auf 120°C erhitzt und 20 Minuten auf dieser Temperatur gehalten. Dann wird das inaktivierte Abwasser auf 80°C abgekühlt und danach unter Hinzumischen der zehnfachen Menge Leitungswasser in die Kanalisation gegeben.

### Beispiel 1: Abbau von DNA durch DNasen im Kulturüberstand von animalen Zellen

Während einer Fermentation von Animalzellen sterben kontinuierlich Zellen ab, wodurch Nukleinsäuren und Proteine freigesetzt werden. Bei Produktionsverfahren mit rekombinanter DNA ist es wichtig, während und nach der Fermentation den Verbleib der rekombinanten DNA zu verfolgen. Wir haben gefunden, daß der DNA-Gehalt in Fermentationskulturüberständen der Maus-Zellinie 3T1, welche pCES Plasmid DNA enthält (siehe Patentanmeldung EP-A-267 678) und EPO produziert, sehr gering ist (< 100 pg/ml). Weitere Untersuchungen haben ergeben, daß in Fermentationskulturüberständen dieser Zellen DNase Aktivität vorhanden ist. Die natürliche DNase-Aktivität ist so hoch, daß nach 6 Stunden Inkubation bei Raumtemperatur 10 µg pCES DNS/ml im Kulturüberstand vollständig abgebaut werden kann (Fig.). Deshalb kann man auch in Kulturüberständen mit Hilfe der Southern Blot Hybridisierung weder das 14.3 kb große pCES Plasmidmolekül noch andere kleinere Fragmente des Plasmids nachweisen.

### Beispiel 2: Abbau von DNA durch DNasen im Abwasser

Alle anfallenden Abwässer der vor Beispiel 1 erwähnten Fermentationsanlage werden heutzutage aufgrund gesetzlicher und behördlicher Bestimmungen in einem Abwasser-Auffangbehälter gesammelt und dann in der Inaktivierungsanlage autoklaviert. Dabei wird der Auffangbehälter etwa zu 30% oder mehr gefüllt und danach ca. alle 6 Stunden zum Autoklavieren in die Abtötungsanlage gepumpt. Der Auffangbehälter wird aber nicht vollständig entleert, sondern nur soweit, daß ca. 10 - 20% des Abwassers zurückbleiben. Dieses Abwasser wird dann mit dem neu hinzugefüllten Abwasser vermischt. Ähnliche DNA-Abbauversuche wie mit den Fermentationskulturüberständen (s. Beispiel 1) wurden auch mit Proben aus dem Abwasserauffangbehälter durchgeführt. Auch hier konnten jeweils eine DNase Aktivität von mindestens 10 µg DNA/ml/6h festgestellt werden.

Die Transformierbarkeit von 10 µg Plasmid DNA in kompetente E.coli Bakterien (2 x 10⁸ Klone/µg DNA), die auf diese Weise behandelt wurden, ist drastisch reduziert und kann in Abhängigkeit von der Abwasserzusammensetzung vollkommen eliminiert werden (Tab.).

Die DNase-Aktivität kann experimentell aus Zellen durch Behandlung mit Leitungswasser freigesetzt werden. Dies entspricht etwa den Bedingungen, wenn die Fermentationsgeräte mit Leitungswasser gereinigt werden.

Bei Anwesenheit von 10 mM EDTA kann die DNase-Aktivität im Abwasser inhibiert werden. EDTA fängt unter Chelatbildung zweiwertige Ionen aus der Flüssigkeit, die für die biologische Aktivität der DNasen essentiell sind. Deshalb ist für ausreichende Konzentration von z.B. Mg⁺ und Zn⁺ Sorge zu tragen, falls EDTA anwesend ist.

Da ins Abwasser geringe Mengen Zelldebris gelangen, wurde DNA daraus isoliert und analysiert. Selbst im Debris, wo die DNA durch Zellproteine relativ geschützt ist, konnten nur Bruchstücke, aber keine intakten pCES Plasmidmoleküle nachgewiesen werden.

### Beispiel 3: Isolierung von Mikroorganismen aus Abwasser

Da die Abwässer im Auffangbehälter (s. Beispiel 2) nicht mehr steril sind, wurde untersucht, ob sich im Abwasser Mikroorganismen anreichern können. Es wurde festgestellt, daß die Keimzahl (Kolonie bildende Einheiten) bei Inkubation von 30°C auf LB-Agar bei 10⁵/ml lag; auf YPD-Agar betrug die meßbare Keimzahl 10⁴/ml und auf YNB-Agar 10³.

Die Keimzahlwerte werden im Behälter nach Einfüllen von frischem Abwasser durchaus schwanken, doch zeigen die oben ermittelten Werte, daß das organische Material im Abwasser ausreicht, auch unter schlechten Kulturbedingungen (kein Rühren, keine zusätzliche Belüftung) und trotz Zusatz von Spülmitteln eine beachtliche Vermehrung von Mikroorganismen zu gewährleisten.

Es wurde untersucht, ob diese aus dem Abwasser isolierten Mikroorganismen rekombinante DNA aufnehmen und vermehren können. Das pCES-Plasmid oder ein Fragment davon konnte durch Koloniehybridisierung in keinem der Organismen nachgewiesen werden.

Da durch lysierte Mauszellen rekombinante Nukleinsäure freigesetzt wird, wurde nun versucht, aus dem Abwasser Mikroorganismen zu isolieren, die in der Lage sind, DNasen auszuscheiden. Es wurden Aliquots von 0.01, 0.05 und 0.1 ml Abwasser auf LB-Agar (Ansatz 1) und YPD-Agar (Ansatz 2) ausgespatelt und bei 30°C inkubiert. Außerdem wurde versucht, in einem Medium, das fast ausschließlich DNA als Energie- und Kohlenstoffquelle besitzt, Mikroorganismen mit DNase Aktivität aus dem Abwasser anzureichern. Dazu wurde folgendes Medium angesetzt:

### DNA-Medium:

- 0,1 g: DNA aus Heringssperma
- 19,0 ml: Leitungswasser
- 0,01 ml: LB-Medium
- 0,1 ml: M9 10x Salzlösung
- 0,01 ml: 1 M Cacl₂
- 0,01 ml: 1 M MgCl₂
- 0,01 ml: 1 M (NH₄)₂SO₄

### 10 x M9-Medium:

- 70 g: Na₂HPO₄ x H₂O
- 30 g: KH₂PO₄
- 10 g: NH₄Cl
H₂O ad 1000 ml

### LB-Medium:

- 10 g: Trypton
- 5 g: Hefeextrakt
- 5 g: NaCl
H₂O ad 1000 ml

### YPD-Medium:

- 20 g: Pepton
- 10 g: Hefeextrakt
- 20 g: Glukose
H₂O ad 1000 ml

Dieses DNA-Medium wurde mit 1 ml Abwasser versetzt und vier Tage bei 30°C inkubiert. Das Medium wurde trüb, und mikroskopisch waren verschiedene Mikroorganismen zu identifizieren. Aus diesem Ansatz wurden Aliquots von 0.01 und 0.05 ml entnommen und auf LB-Agar ausgespatelt (Ansatz 3). In allen Ansätzen konnten Mikroorganismen isoliert und in Reinkultur genommen werden.
Ansatz 1 (LB-Agar): AW1, AW2, AW3, AW4, AW5, AW8, AW9
Ansatz 2 (YPD-Agar): AW10, AW11, AW12, AW13
Ansatz 3 (DNA-Medium): AW6, AW7

### Beispiel 4: Charakterisierung von sieben in Reinkultur genommenen Mikroorganismen

Dreizehn Mikroorganismen mit unterschiedlicher Koloniemorphologie und Farbe wurden in Reinkultur genommen. Sie wuchsen alle auf Agar-Platten mit YPD oder LB-Medium. Das Isolat AW10, ein Schimmel mit schwarzem Luftmycel, wurde nicht näher untersucht, weil es in YPD und LB-Flüssigkulturen nicht wuchs. Die Isolate AW1, AW2, AW4, AW6 und AW12 wurden verworfen, da sie nach ersten Untersuchungen bei Wachstum in verschiedenen Medien anscheinend keine DNasen ausscheiden. Das Isolat AW3 sekretiert in Flüssigkulturen ebenfalls keine DNasen, wurde aber in weiteren Versuchen als Negativkontrolle mitgeführt.

Beschreibung der Kolonien der Isolate bei Wachstum auf LB-Agar:
- AW3:: gelbliche Färbung
- AW5:: farblos
- AW6:: pilzartiges Erscheinungsbild, gefranster Rand, Kolonie erhaben, beigefarben
- AW7:: weiß, glänzend
- AW8:: orangefarben
- AW11:: beigefarben
- AW13:: weißes Luftmycel, nach einer Woche grau werdend, über die gesamte Platte wachsend (hier Analyse auf YPD-Medium)

Lichtmikroskopische Beschreibung der Isolate bei Wachstum in YPD-Medium bei 30°C:
- AW3:: Bakterium, coccoid, Zellen aggregieren zu Haufen
- AW5:: Bakterium, coccoid, Zellen als Diplo- oder Streptococcen wachsend
- AW6:: Hefeartige Zellen, rund bis leicht oval, teilweise kleine Ketten bildend, auch verzweigte Formen
- AW7:: Bakterium, coccoid, im Verband wachsend, größer als AW3 und AW5
- AW8:: Bakterium, coccoide bis ovale Zellen, im Verband wachsend
- AW11:: Hefeartige große Zellen, rund, einzeln oder paarig, keine Verzweigungen
- AW13:: Schimmelpilz mit weißem Luftmycel auf Agar-Platten, verzweigtes Mycel, sehr dicht in YPD wachsend

### Beispiel 5: Nachweis sekretierter DNasen

Es wurden 30 ml LB-Medium bzw. YPD-Medium in 300 ml-Erlenmeyerkolben mit acht der Isolate angeimpft und bei 30°C bei 120 Upm inkubiert. Nach 70 Stunden Inkubation wurden die Zellen abzentrifugiert und der Überstand auf DNase-Aktivität getestet.

### Versuch A:

- 0.08 ml: Kultur-Überstand
- 0.01 ml: Puffer (500 mM Tris-HCl, pH 7,5; 50 mM MgCl₂)
- 0.01 ml: Plasmid-Lösung (1.74 mg/ml)

### Versuch B:

- 0.08 ml: Kultur-Überstand
- 0.01 ml: Puffer (500 mM Tris-HCl, pH 7,5; 50 mM MgCl₂)
- 0.015 ml: lambda DNA (0,5 mg/ml)

Nach 0, 6, 10 und 16 Stunden wurde jeweils 0.03 ml entnommen, mit 0,005 ml STOP-Mix versetzt (100 mM EDTA, 20% Saccharose, Bromphenolblau als Marker), bei -20°C eingefroren und anschließend in einem 0.8% Agarosegel bei 90 V in 3 Stunden aufgetrennt (Versuch A). Beim Versuch B wurden nach 0, 8 und 24 Stunden Aliquots zur Analyse entnommen.

Extrazelluläre DNase Aktivität war in Kulturüberständen der Isolate AW5, AW6, AW7, AW8, AW11 und AW13 nachweisbar. Die DNase Aktivität unterschied sich zum Teil bei Anzucht auf LB-Medium oder YPD-Medium. So schied z.B. das Isolat AW6 mehr DNasen bei Anzucht auf YPD-Medium als bei Anzucht auf LB-Medium aus. Das Isolat AW8 hingegen sekretierte mehr DNasen bei Wachstum auf LB-Medium. Beim Isolat AW3 war andererseits weder bei Wachstum in LB-Medium noch YPD-Medium unter den angegebenen Versuchsbedingungen DNase Akitivität nachweisbar; die höchste DNase-Aktivität wurde unter den gegebenen Bedingungen vom Isolat AW13 nachgewiesen. Dieser Organismus schied in LB-und YPD-Medium reichlich DNase(n) aus, die sowohl zirkuläre Plasmid-DNA als auch lineare lambda DNA in kurzer Zeit total abbauen konnte(n).

### Beispiel 6: Sekretion von DNasen durch das Isolat AW13 in Abwasser

Das Isolat AW13 bildet nicht nur bei Wachstum in YPD und LB-Medium DNasen, sondern auch im Abwasser, wie es bei der Produktion von EPO anfällt.

300 ml Abwasser wurden 20 Minuten bei 121°C autoklaviert und mit 50 µl einer 2 Tage alten AW13-Kultur (gewachsen in YPD) angeimpft. Nach 24 Stunden und nach 96 Stunden wurden 1 ml Kulturbrühe entnommen, abzentrifugiert und der überstand auf DNase-Aktivität mit Plasmid-DNA und lambda-DNA als Substrat überprüft. Der Versuchsansatz erfolgte wie unter Beispiel 5 beschrieben. Die Probeentnahme erfolgte nach 0 Stunden, 3,5 Stunden und 7 Stunden. Als Kontrolle wurde DNA in autoklaviertem Abwasser inkubiert. Abwasser, in dem AW13 gewachsen war, enthielt hohe DNase-Aktivitäten. Sowohl Plasmid als auch lambda-DNA wurden degradiert.

**Tab.**

| Transformation von 100pg DNA Äquivalenten nach Inkubation von 10 µg pCES DNA bei Raumtemperatur in Abwässern aus einer Fermentationsanlage für Animalzellen. | | | | |
|---|---|---|---|---|
| **Inkubationsbedingungen** | **Ausgezählte Kolonien auf Ampicillin Platten** | | | |
| | Abwasserprobe (Nr.) | | | |
| | 1 | 2 | 3 | 4 |
| 1 Minute ohne EDTA | 6100 | 13300 | 10800 | 9600 |
| 1 Minute mit EDTA | 6742 | 10020 | 9600 | 7200 |
| 6 Stunden ohne EDTA | 10 | 2 | 134 | 0 |
| 6 Stunden mit EDTA | 6100 | 6000 | 9000 | 6100 |

Jeweils 10 µg pCES Plasmid DNA wurde in 1 ml Abwasser gegeben und für 1 Minute bzw. 6 Stunden bei Raumtemperatur mit oder ohne EDTA (2mM) inkubiert. Danach wurden 10 µl entnommen und in kompetente Bakterien des Stammes E.coli DH5 transformiert. Die verschiedenen Proben (1-4) entsprechen Abwasserentnahmen aus dem Auffangbehälter und mit unterschiedlichen Füllhöhen und verschiedenen Abwasserzusammensetzungen.

### Legende zu Fig.:

Jeweils 10 µg pCES DNA wurden in 1 ml Abwasser aus dem Auffangbehälter unterschiedlich lange inkubiert. Danach wurden 10 µl entnommen und auf einem Agarosegel aufgetrennt. Das Gel wurde auf Nitrocellulose geblottet und gegen ein radioaktiv markiertes Fragment von 800 Basenpaaren (bp) aus dem pCES Plasmid hybridisiert und exponiert. Die Signale auf dem Röntgenfilm wurden quantitativ ausgewertet und gegen die Inkubationszeit aufgetragen.

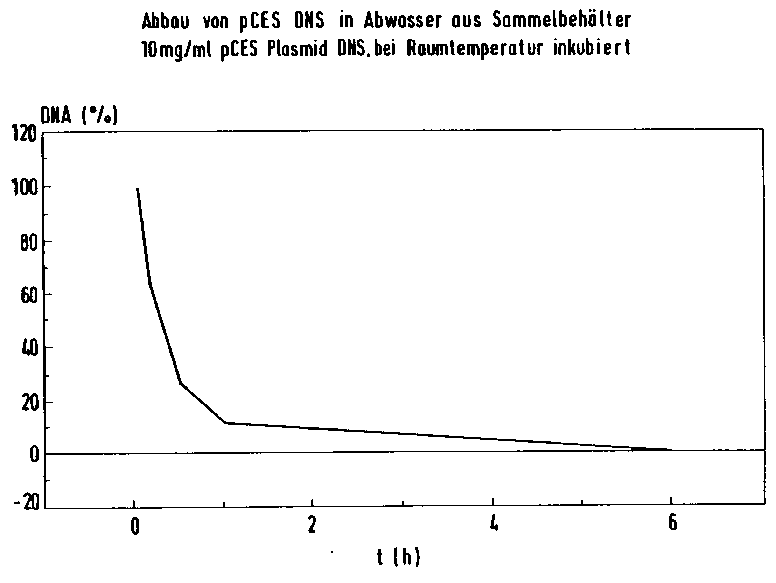

## Patentansprüche

1. Verwendung von Paecilomyces lilacinus-Stämmen, die Nukleasen ausscheiden, zum Abbau von Nukleinsäuren.

2. Verwendung der von Paecilomyces lilacinus-Stämmen ausgeschiedenen Nukleasen zum Abbau von Nukleinsäuren.

3. Verwendung nach Anspruch 1 oder 2, dadurch gekennzeichnet, daß Nukleinsäuren in mit gentechnisch manipulierten Mikroorganismen kontaminiertem Material abgebaut werden.

4. Mikroorganismus AW13, hinterlegt unter Nr. DSM 5650 bei der Deutschen Sammlung von Mikroorganismen.

## Claims

1. The use of Paecilomyces lilacinus strains which excrete nucleases for breaking down nucleic acids.

2. The use the nucleases excreted by Paecilomyces lilacinus strains for breaking down nucleic acids.

3. The use as claimed in claim 1 or 2, wherein nucleic acids are broken down in material contaminated with genetically manipulated microorganisms.

4. The microorganism AW13, deposited under No. DSM 5650 at the German Microorganism Collection.

## Revendications

1. Utilisation de souches de *Paecilomyces* lilacinus, qui produisent des nucléases, pour la dégradation d'acides nucléiques.

2. Utilisation des nucléases produites par des souches *Paecilomyces lilacinus* pour la dégradation d'acides nucléiques.

3. Utilisation selon la revendication 1 ou 2, caractérisée en ce que l'on dégrade des acides nucléiques d'un matériel contaminé par des micro-organismes manipulés par génie génétique.

4. Microorganisme AW13, déposé sous le numéro DSM 5650 à la Collection Allemande de Micro-organismes.
